# DEMANDE DE BREVET EUROPEEN

(11) **EP 3 488 810 A1**
(43) Date de publication de la demande: **29.05.2019**
(21) Numéro de dépôt: 18207178.7
(22) Date de dépôt: 20.11.2018
(51) Int. Cl.: A61B 90/00

(54) **DISPOSITIF D'ANCRAGE ET DE REPERAGE DE LESIONS SUSPECTES A MULTIPLES ORGANES D'ANCRAGE**

(30) Priorité: 22.11.2017 FR 1761057
(71) Demandeur: Fumex, Laurent, Madison, CT 06443 (US); Masseglia, Thierry, 83130 La Garde (FR)
(72) Inventeur: Fumex, Laurent, Madison, CT 06443 (US); Masseglia, Thierry, 83130 La Garde (FR)
(74) Mandataire: Tessier, Benoit Sebastien

(57) **Abrégé**

L'invention concerne un dispositif d'ancrage (4) pour le repérage de lésion ou de microcalcification dans un tissu biologique, comprenant :
- un fil guide (7) comportant au moins une portion graduée pour la détermination de la profondeur d'ancrage du dispositif d'ancrage,
- au moins deux organes d'ancrage (5) dans un tissu biologique raccordés au fil guide (7), et
- une bague de raccordement (6) du fil guide (7) aux organes d'ancrage (5).

L'invention concerne également un système d'ancrage comprenant un tel dispositif d'ancrage (4), un mandrin et une aiguille creuse de positionnement.

## Description

La présente invention concerne un dispositif en radiologie et en chirurgie, et plus particulièrement un dispositif d'ancrage et de repérage de nodules pulmonaires en vue de leur ablation par voie chirurgicale, ainsi qu'un appareil de mise en place.

Le cancer du poumon est un cancer affectant un nombre important de patients et reflétant le plus haut taux de mortalité. Le recours à la biopsie en vue d'un diagnostic histologique est donc devenu une procédure indispensable et ce d'autant plus que le taux de malignité des nodules pulmonaires est très élevé. Les biopsies sont traditionnellement réalisées par voie trans-bronchique ou par voie percutanée à l'aide d'aiguilles fines sous scanner. Cependant le dépistage de plus en plus précoce des nodules et leur géographie peuvent limiter ces techniques traditionnelles de biopsie rendant de facto nécessaire l'intervention chirurgicale.

Lorsque la chirurgie est possible, il existe deux techniques, la thoracotomie et la thoracoscopie.

La thoracotomie consiste en une incision chirurgicale de la paroi thoracique. La chirurgie peut nécessiter l'ouverture du thorax ou simplement une incision entre les côtes. Cette chirurgie dite « ouverte » est invasive et lourde à supporter pour le patient.

La thoracoscopie est une chirurgie minimalement invasive du thorax. C'est une chirurgie dite « vidéo-assistée » impliquant la réalisation de plusieurs petites incisions (d'environ 2 cm de long) à l'aide d'instruments longs et fins. La lumière et la vision sont fournies par un dispositif optique introduit à travers l'une de ces incisions. Cette dernière technique est donc très avantageuse mais exige nécessairement à titre préalable de localiser et de repérer le ou les nodules qui doivent être enlevés.

Le repérage des nodules est ainsi confié préalablement à l'acte chirurgical à des radiologues, lesquels ont recours à des techniques de repérage préopératoire utilisant le plus souvent un harpon placé, sous contrôle scanographique, à proximité du nodule à enlever.

Actuellement, la plupart des harpons utilisés lors d'une procédure de repérage de nodules pulmonaires ont été conçus pour le repérage des lésions et microcalcifications du sein et ne sont pas adaptés pour une utilisation en thoracoscopie. En effet pour être mis en place par le radiologue en toute sécurité sous contrôle scanographique et servir de repérage fiable au chirurgien lors de l'ablation, le système de repérage doit tout d'abord comporter un appareil de mise en place dont le diamètre externe de l'aiguille est adapté aux contraintes chirurgicales, de préférence inférieur 1,25mm (18 gauges), ceci en particulier afin de réduire les risques de pneumothorax ; le système doit ensuite pouvoir s'adapter aux mouvements d'amplitude induits par le gonflement et le dégonflement du poumon et de la cage thoracique durant les phases de ventilation imposées par la thoracoscopie ; ceci afin d'assurer la pérennité de l'ancrage dans les tissus durant toute la procédure. Enfin, le dispositif doit offrir une exceptionnelle qualité d'ancrage dans les tissus pulmonaires dont la composition et la texture même rend difficile toute accroche, cela afin d'éviter tout risque de migration du dispositif et donc de perte de repérage avant et pendant la chirurgie.

Un exemple d'un dispositif d'ancrage connu est décrit dans le document FR 2 731 343 A. Ce dispositif d'ancrage comprend un manchon dont une extrémité est solidaire d'un fil et une autre extrémité opposée est solidaire d'un brin de section circulaire ou rectangulaire adapté à s'ancrer dans les tissus mammaires pour repérer un nodule. Pour solidariser le brin à l'intérieur du manchon, Le brin est serti à l'intérieur du manchon de manière à les solidariser l'un et l'autre. Le dispositif d'ancrage peut comprendre plusieurs brins sertis à l'intérieur du manchon. Dans ce dernier cas, les dimensions du manchon doivent être adaptées pour accueillir l'ensemble des brins. Tel qu'indiqué ci-avant, un tel dispositif d'ancrage n'est toutefois pas adapté pour une utilisation en thoracoscopie. En effet, il a été constaté qu'en utilisant un tel dispositif d'ancrage ayant des dimensions adaptées à la thoracoscopie, c'est-à-dire lorsqu'il ne comprend qu'un seul brin, sa capacité d'ancrage est trop faible de sorte que le dispositif d'ancrage a tendance à migrer.

Il existe donc un besoin pour un dispositif d'ancrage et de repérage de nodules pulmonaires en vue de leur ablation par voie chirurgicale ayant des dimensions adaptées à la thoracoscopie, capable en outre d'absorber les amplitudes liées à la thoracoscopie et enfin ayant une capacité d'ancrage adapté à la structure des tissus pulmonaires permettant d'éviter la migration du dispositif d'ancrage avant et pendant la chirurgie.

Pour cela, l'invention concerne un dispositif d'ancrage pour le repérage de lésion ou de microcalcification dans un tissu biologique, comprenant :
- un fil guide,
- au moins deux organes d'ancrage dans un tissu biologique raccordés au fil guide, et
- une bague de raccordement du fil guide aux organes d'ancrage, dans lequel chaque organe d'ancrage comporte une extrémité de raccordement s'étendant le long d'un axe longitudinal et configurée pour être disposée à l'intérieur de la bague de raccordement, chaque extrémité de raccordement ayant une section transversale formant un secteur angulaire, la somme des secteurs angulaires étant égale ou inférieure à 360° dans un même plan transversal à la l'axe longitudinal.

Cette configuration du dispositif d'ancrage permet de réaliser le raccordement d'une pluralité d'organes d'ancrage à l'intérieur d'une bague de raccordement ayant des dimensions semblables à celles utilisées lorsque le dispositif d'ancrage comprend un seul organe d'ancrage. En d'autres termes, cette configuration permet de réduire l'encombrement des organes d'ancrage à l'intérieur de la bague de raccordement. Or, augmenter le nombre d'organes d'ancrage permet d'améliorer la capacité d'ancrage du dispositif. Ainsi, la capacité d'ancrage du dispositif d'ancrage peut être améliorée tout en ayant un dispositif d'ancrage dont les dimensions sont conformes à la thoracoscopie.

Selon un mode de réalisation du dispositif d'ancrage, les extrémités de raccordement sont complémentaires les unes des autres de manière à former une section circulaire lorsque les extrémités de raccordement sont disposées en contact les unes par rapport aux autres.

Selon un mode de réalisation du dispositif d'ancrage, chaque extrémité de raccordement comporte une surface extérieure au contact de la bague de raccordement et au moins une surface intérieure disposée au contact d'au moins une autre extrémité de raccordement.

Selon un mode de réalisation du dispositif d'ancrage, la surface extérieure est courbe et la surface intérieure est plane.

Selon un mode de réalisation du dispositif d'ancrage, les organes d'ancrage comprennent une portion d'ancrage formée dans le prolongement de l'extrémité de raccordement, la portion d'ancrage comprenant au moins une surface plane s'étendant transversalement à l'axe longitudinal.

Selon un mode de réalisation du dispositif d'ancrage, les organes d'ancrage comprennent au niveau d'une extrémité opposée à l'extrémité de raccordement un bord d'attaque du tissu biologique formé par au moins une facette.

Selon un mode de réalisation du dispositif d'ancrage, chaque organe d'ancrage est configuré pour être disposé dans :
- une première position dans laquelle l'organe d'ancrage a une configuration permettant d'insérer l'organe d'ancrage dans un tissu biologique, et
- une deuxième position dans laquelle l'organe d'ancrage a une configuration permettant de retenir l'organe d'ancrage dans un tissu biologique.

Selon un mode de réalisation du dispositif d'ancrage, les organes d'ancrage sont configurés pour s'étendre dans des directions différentes les uns des autres lorsque les organes d'ancrage sont chacun disposés dans leur deuxième position.

Selon un mode de réalisation du dispositif d'ancrage, les organes d'ancrage sont réalisés chacun dans un matériau élastique permettant aux organes d'ancrage d'être déformés entre les première et deuxième positions.

Selon un mode de réalisation du dispositif d'ancrage, celui-ci comprenant en outre une butée formant un appui pour l'entrainement longitudinal du dispositif d'ancrage.

Selon un mode de réalisation du dispositif d'ancrage, le fil guide comporte au moins une portion graduée pour la détermination de la profondeur d'ancrage du dispositif d'ancrage.

Selon un mode de réalisation du dispositif d'ancrage, la portion graduée est plane.

Selon un mode de réalisation du dispositif d'ancrage, la portion graduée comprend un élargissement local de la section transversale du fil formant la butée.

Selon un mode de réalisation du dispositif d'ancrage, la portion graduée est disposée à une longueur prédéterminée de la bague de raccordement.

L'invention concerne également un système d'ancrage pour le repérage de lésion ou de microcalcification dans un tissu biologique, comprenant :
- une aiguille creuse de positionnement,
- un dispositif d'ancrage selon l'une quelconque des revendications précédentes configuré pour coulisser à l'intérieur de l'aiguille creuse de positionnement,
- un mandrin configuré pour coulisser à l'intérieur de l'aiguille creuse de positionnement de manière à entrainer les organes d'ancrage hors de l'aiguille creuse de positionnement.

Selon un mode de réalisation du système d'ancrage, l'un au moins parmi les deux organes d'ancrage du dispositif d'ancrage comporte une section transversale égale ou inférieure au demi-diamètre interne de l'aiguille creuse de positionnement.

Selon un mode de réalisation du système d'ancrage, le mandrin comporte un trou traversant permettant au mandrin de coulisser autour et le long du fil guide du dispositif d'ancrage.

Selon un mode de réalisation du système d'ancrage, le mandrin est configuré pour entrainer les organes d'ancrage en prenant appui sur la butée.

Selon un mode de réalisation du système d'ancrage, l'aiguille creuse de positionnement est configurée pour disposer les organes d'ancrage dans la première position lorsque les organes d'ancrage sont disposés à l'intérieur de l'aiguille creuse de positionnement, les organes d'ancrage étant configurés pour se déformer en vue d'adopter la deuxième position une fois entrainés hors de l'aiguille creuse de positionnement.

D'autres caractéristiques et avantages de l'invention apparaîtront à la lecture de la description qui suit de modes de réalisation préférés de l'invention, donnée à titre d'exemple et en référence aux dessins annexés.
La figure 1 représente une vue en perspective d'un système d'ancrage comprenant un mandrin et un dispositif d'ancrage disposés à l'intérieur d'une aiguille creuse.
La figure 2 représente une vue en perspective du mandrin hors de l'aiguille creuse.
La figure 3 représente une vue de face du dispositif d'ancrage de la figure 1 hors de l'aiguille creuse.
Les figures 4 et 5 représentent une vue en perspective d'un mode de réalisation d'un organe d'ancrage du dispositif d'ancrage des figures 1 et 3.
La figure 6 représente une vue en perspective d'un autre mode de réalisation d'un organe d'ancrage du dispositif d'ancrage des figures 1 et 3.

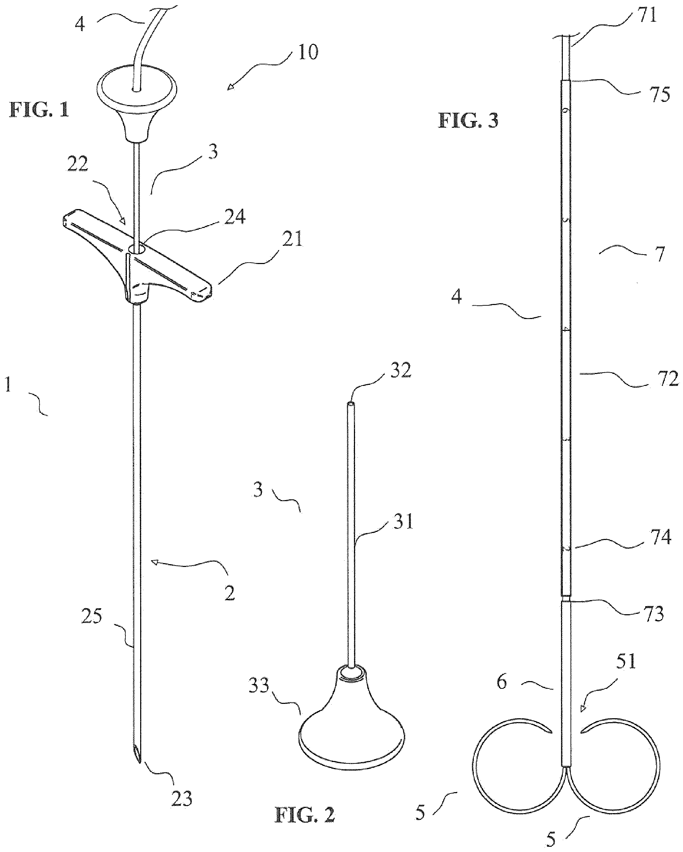

Tel que représenté sur la figure 1, un système d'ancrage 10 pour le repérage de lésion ou de microcalcification dans un tissu biologique comprend une aiguille creuse de positionnement 2 et un dispositif d'ancrage 4 configuré pour coulisser à l'intérieur de l'aiguille creuse de positionnement 2. Le système d'ancrage 10 comprend également un mandrin 3 configuré pour coulisser à l'intérieur de l'aiguille creuse de positionnement 2 de manière à entrainer le déplacement du dispositif d'ancrage 4 hors de l'aiguille creuse de positionnement 2. Le déplacement du dispositif d'ancrage 4 par le mandrin 3 se fait par coulissement le long de l'aiguille creuse de positionnement 2.

Pour permettre le coulissement du mandrin 3 et du dispositif d'ancrage 4 à l'intérieur de l'aiguille creuse de positionnement 2, celle dernière forme un trou traversant 22 s'étendant longitudinalement. L'aiguille creuse de positionnement 2 comporte une extrémité de perforation 23 configurée pour perforer un ou plusieurs types de tissus. En particulier, l'extrémité de perforation 23 est biseautée de manière à former une pointe apte à perforer les tissus. L'extrémité de perforation 23 constitue une extrémité d'entrée du dispositif d'ancrage 4 lors de son montage dans l'aiguille creuse de positionnement 2 et de sortie du dispositif d'ancrage 4 de l'aiguille creuse de positionnement 2 lors de son entrainement par le mandrin 3. L'aiguille creuse de positionnement 2 comprend également une extrémité d'insertion 24 du mandrin 3. L'extrémité d'insertion 24 est opposée à l'extrémité de perforation 23. Le trou traversant 22 s'étend entre les extrémités de perforation 23 et d'insertion 24. L'aiguille creuse de positionnement 2 s'étend de préférence sur une longueur entre 100mm et 150mm. Le diamètre interne de l'aiguille creuse de positionnement 2 est de préférence égal ou inférieur à 0,95mm. Pour la manipulation du système d'ancrage 10, l'aiguille creuse de positionnement 2 comprend également une poignée 21 disposée au niveau de l'extrémité d'insertion 24. La poignée 21 est configurée pour recevoir, de part et d'autre de la poignée, deux doigts d'un utilisateur pour réaliser un effort de retenue de l'aiguille creuse de positionnement 2 lorsque le mandrin 3 est poussé à l'intérieur de l'aiguille creuse de positionnement 2. De préférence, l'utilisateur place son index et son majeur sous la poignée 21 de manière à actionner le mandrin 3 avec son pouce, telle qu'une seringue. La poignée 21 est par exemple surmoulée autour d'un corps d'aiguille 25 formant le trou traversant 22. De préférence, la poignée 21 est en matériau plastique et le corps d'aiguille 25 en matériau métallique.

Le corps d'aiguille 25 est de préférence de section circulaire. De manière alternative, le corps d'aiguille 25 peut être de toute section, par exemple rectangulaire, carrée ou ovale.

Tel que représenté sur la figure 2, le mandrin 3 comprend un corps de mandrin 31 et un poussoir 33 monté sur le corps de mandrin 31. Le poussoir 33 est de préférence surmoulé sur le corps de mandrin 31. De préférence encore, le corps de mandrin 31 est réalisé en matériau métallique et le poussoir 33 est réalisé en matériau plastique. Le poussoir 33 est configuré de sorte que le doigt d'un utilisateur puisse agir sur une portion distale du poussoir 33 de manière à entrainer le dispositif d'ancrage 4. Ainsi, en combinaison avec la poignée 21, le système d'ancrage 10 peut être utilisé seulement à l'aide de trois doigts, de préférence le majeur, l'index et le pouce, telle qu'une seringue. Le système d'ancrage 10 est ainsi simple d'utilisation car configuré pour être utilisé seulement à l'aide d'une main.

Le mandrin 3 comprend également une extrémité d'entrainement 32 apte à coopérer avec une butée du dispositif d'ancrage 4 pour entrainer le coulissement du dispositif d'ancrage 4 à l'intérieur de l'aiguille creuse de positionnement 2.

Tel que représenté sur la figure 3, le dispositif d'ancrage 4 comprend également un fil guide 7 et deux organes d'ancrage 5 dans un tissu biologique raccordés au fil guide 7. Les deux organes d'ancrage 5 sont assemblés à une extrémité du fil guide 7 à l'aide d'une une bague de raccordement 6. En particulier, une extrémité du fil guide 7 est sertie à l'intérieur d'une extrémité de la bague de raccordement 6. De manière similaire une extrémité de chacun des organes d'ancrage 5 est sertie à l'intérieur de la bague au niveau d'une extrémité de la bague de raccordement 6 à l'opposé du fil guide 7. Ainsi, le fil guide 7, la bague de raccordement 6 et les organes d'ancrage 5 forment un ensemble solidaire.

Cet ensemble solidaire formé par le fil guide 7, la bague de raccordement 6 et les organes d'ancrage 5 est destiné à être inséré à l'intérieur de l'aiguille creuse de positionnement 2 et du mandrin 3 pour être introduit dans les tissus d'un patient. De manière alternative, le fil guide 7, la bague de raccordement 6 et les organes d'ancrage 5 peuvent être assemblés entre eux par tout moyen permettant à cet ensemble solidaire d'être inséré à l'intérieur du mandrin 3.

Le fil guide 7 comporte au moins trois portions, une portion proximale 73 destinée à être sertie dans la bague de raccordement 6, la portion graduée 72 configurée pour le marquage centimétrique et dont le diamètre externe est supérieur au diamètre externe de la portion proximale 73 et supérieur au diamètre interne du corps du mandrin 31, ainsi qu'une portion distale 71 configurée pour coulisser dans le corps du mandrin 31. Le fil guide 7 correspond de préférence à un fil souple dont une portion intermédiaire entre ses extrémités est aplatie pour former la portion graduée 72. On entend par « souple » le fait que le fil peut être enroulé sur lui-même de manière à former une boucle d'un diamètre inférieur à 10cm, de préférence inférieur à 6cm, sans déformation plastique du fil. A titre d'exemple, le fil souple peut être en matériau polymère, tel que le polyamide biocompatible. Le fil peut également être métallique et tressé, i.e. composé de plusieurs brins enroulés les uns avec les autres. Parmi les trois portions du fil guide 7, au moins la portion graduée 72 du fil guide 7 est souple. A titre de comparaison, un fil « rigide » correspond à un fil plein métallique de même diamètre externe.

Le fil guide 7 comprend au moins une portion graduée 72 pour la détermination de la profondeur d'ancrage du dispositif d'ancrage 4. Pour cela, ladite portion graduée comprend des graduations 74, par exemple centimétriques, formée sur la portion graduée 72. De plus, la portion graduée 72 est disposée à une longueur prédéterminée de la bague de raccordement 6. Lorsque le dispositif d'ancrage 4 est disposé à l'intérieur des tissus biologiques, le fil guide 7 s'étend hors de l'incision faite pour l'insertion du dispositif d'ancrage 4. En particulier, le fil guide 7 est configuré de sorte que la portion graduée 72 dépasse de cette incision de sorte que le chirurgien est capable de connaître la profondeur d'ancrage du dispositif d'ancrage 4. De préférence, la portion graduée 72 est plane ou comprend une surface graduée plane pour faciliter la réalisation des graduation 74. La portion graduée 72 est de préférence réalisée par un aplatissement localisé du fil guide 7 pour obtenir une portion graduée 72 plane. Le fil guide 7 peut comprendre une pluralité de portions graduées 72 pour former une zone de graduation discontinue. Dans ce dernier cas, chaque portion graduée 72 peut comprendre une ou plusieurs graduations 74 tout en conservant une distance prédéfinie entre chaque graduation 74.

Le fil guide 7 comprend également une butée 75 formant un appui pour l'entrainement longitudinal du dispositif d'ancrage 4. La butée 75 forme une excroissance transversale à la direction d'extension du fil guide 7. Lorsque le dispositif d'ancrage 4 est disposé à l'intérieur de l'aiguille creuse de positionnement 2 avec le mandrin 3 monté sur la portion distale 71 du fil guide 7, cette excroissance est configurée pour former une butée longitudinale d'appui pour l'extrémité d'entraînement 32 de manière à entrainer le dispositif d'ancrage 4 à l'intérieur de l'aiguille creuse de positionnement 2. Ainsi, le mandrin 3 est de préférence disposé autour du fil guide 7 dans une partie supérieure du fil guide 7 disposée au-dessus de la butée 75.

La butée 75 est de préférence réalisée par la portion graduée 72. En effet, la portion graduée 72 peut comprendre un élargissement local de la section transversale du fil guide 7 formant la butée 75. La butée 75 est de préférence réalisée par une extrémité de la portion graduée 72. Lorsque le fil guide 7 est aplati, une augmentation locale d'une dimension transversale du fil guide 7 est générée pour former la butée 75. Pour permettre à la butée 75 de réaliser sa fonction de butée d'appui, cette dimension transversale à la direction d'extension du fil est supérieure au diamètre interne du corps de mandrin 31. Pour le coulissement du dispositif d'ancrage 4 à l'intérieur de l'aiguille creuse de positionnement 2, cette dimension transversale est par ailleurs inférieure au diamètre interne du corps d'aiguille 25.

Le fil guide 7 est réalisé dans un matériau souple utilisé pour les fils à suture, de préférence en polyamide biocompatible. Ainsi, le fil guide 7 permet de ne pas blesser le patient pendant sa respiration et de n'exercer aucun effort sur le dispositif d'ancrage 4 et donc de réduire les risques de migration. La réalisation d'une portion graduée 72 dans le matériau du fil guide 7 permet de conserver ses avantages tout en fournissant l'information au chirurgien de la profondeur d'ancrage.

Les dimensions du mandrin 3 sont configurées pour permettre au mandrin 3 d'être disposé à l'intérieur de l'aiguille creuse de positionnement 2, entre le dispositif d'ancrage 4 et l'aiguille creuse de positionnement 2. Pour cela, le diamètre externe du corps de mandrin 31 est inférieur au diamètre interne du corps d'aiguille 25 et le diamètre interne du corps de mandrin 31 est supérieur au diamètre externe de la portion distale 71 du fil guide 7 du dispositif d'ancrage 4 tout en étant inférieur à la section transversale de la butée 75 du fil guide 7. Le corps de mandrin 31 est de préférence de section circulaire. De manière alternative, le corps de mandrin 31 peut être de toute section permettant au mandrin 3 de coulisser à l'intérieur de l'aiguille creuse de positionnement 2 et à la portion distale 71 du fil guide 7 du dispositif d'ancrage 4 de coulisser à l'intérieur du mandrin 3.

Chaque organe d'ancrage 5 est configuré pour être disposé dans une première ou une deuxième position. Dans la première position, l'organe d'ancrage 5 a une configuration permettant d'insérer l'organe d'ancrage 5 dans un tissu biologique. Autrement dit, l'organe d'ancrage 5 est configuré pour être disposé dans une position étendue permettant son insertion à l'intérieur de l'aiguille creuse de positionnement 2. L'aiguille creuse de positionnement 2, peut donc être insérée à l'intérieur des tissus biologiques du patient avec l'organe d'ancrage 5 disposé en son sein. Dans cette première position, l'organe d'ancrage 5 ne perfore pas lui-même les tissus biologiques du patient. La première position peut ainsi être qualifiée d'inactive ou de position de non-retenue des tissus biologiques. Dans la deuxième position, l'organe d'ancrage 5 a une configuration permettant de retenir l'organe d'ancrage 5 dans un tissu biologique. En d'autres termes, l'organe d'ancrage 5 est configuré pour être disposé dans une position de retenue apte à se mettre en opposition par rapport à des tissus biologiques dans lesquels il est inséré. Dans cette position de retenue, l'organe d'ancrage 5 peut prendre la forme d'un crochet. La deuxième position peut donc être qualifiée d'active ou position de retenue des tissus biologiques du patient. En conséquence, chaque organe d'ancrage 5 est configuré pour être disposé en position étendue à l'intérieur de l'aiguille creuse de positionnement 2 avant ancrage et en position de retenue lorsque le dispositif d'ancrage 4 est entrainé par le mandrin 3 à l'extérieur de l'aiguille creuse de positionnement 2. Ainsi, lorsque l'aiguille creuse de positionnement 2 est insérée à l'intérieur du corps d'un patient, l'actionnement du mandrin 3 permet de déployer les organes d'ancrage 5 hors de l'aiguille creuse de positionnement 2 pour les ancrer dans les tissus biologiques. Autrement dit, l'aiguille creuse de positionnement 2 est configurée pour disposer les organes d'ancrage 5 dans la première position lorsque les organes d'ancrage 5 sont disposés à l'intérieur de l'aiguille creuse de positionnement 2, les organes d'ancrage 5 étant configurés pour se déformer en vue d'adopter la deuxième position une fois entrainés hors de l'aiguille creuse de positionnement 2.

Selon une configuration préférée, l'organe d'ancrage 5 comprend une extrémité libre configurée pour perforer les tissus biologiques. En d'autres termes, l'organe d'ancrage 5 présente un profil ouvert avec extrémité proximale solidaire du fil guide 7 et une extrémité distale de perforation des tissus biologiques. Ainsi, lorsque l'organe d'ancrage 5 est mû de la première vers la deuxième positions, l'extrémité distale libre permet à l'organe d'ancrage 5 de perforer les tissus biologiques et de s'y maintenir une fois dans la deuxième position. Cette configuration avec une extrémité libre permet à l'organe d'ancrage 5 de s'ancrer dans une portion de tissu biologique sans avoir à la contourner ou la pincer.

Pour améliorer l'ancrage des organes d'ancrage 5, ceux-ci peuvent être configurés pour s'étendre dans des directions différentes les uns des autres lorsque les organes d'ancrage 5 sont chacun disposés dans leur deuxième position. Les organes d'ancrage 5 sont réalisés de préférence chacun dans un matériau élastique permettant aux organes d'ancrage 5 d'être déformés entre les première et deuxième positions. De manière encore préférée, les organes d'ancrage 5 sont réalisés en polymère à mémoire de forme, en alliage organique ou métallique à mémoire de forme, en polyéthylène ou en alliage nickel/titane.

Chaque organe d'ancrage 5 comporte une extrémité de raccordement 51 s'étendant le long d'un axe longitudinal A et configurée pour être disposée à l'intérieur de la bague de raccordement 6. Lorsque les extrémités de raccordement 51 sont disposées à l'intérieur de la bague de raccordement 6 et que le dispositif d'ancrage est disposé à l'intérieur de l'aiguille creuse de positionnement 2, l'axe longitudinal A est confondu avec l'axe d'extension de de l'aiguille creuse de positionnement 2. De plus, chaque extrémité de raccordement 51 a une section transversale formant un secteur angulaire, la somme des secteurs angulaires étant égale ou inférieure à 360° dans un même plan transversal à la l'axe longitudinal A. En d'autres termes, chaque extrémité de raccordement 51 occupe seulement une portion de l'espace intérieur de la bague de raccordement 6, dans un plan transversal à l'axe longitudinal A. Ainsi, il est possible d'occuper un maximum d'espace à l'intérieur de la bague de raccordement 6 de sorte que le maintien de chacun des organes d'ancrage 5 peut être accompli efficacement. Cette configuration permet de réduire l'encombrement des organes d'ancrage 5 à l'intérieur de la bague de raccordement 6. Ceci est particulièrement utile car les dimensions radiales de la bague de raccordement 6 sont contraintes par l'utilisation du dispositif d'ancrage 4 en thoracoscopie. Ainsi il est possible d'améliorer la fixation d'une pluralité d'organes d'ancrage 5 à l'intérieur de la bague de raccordement 6.

De plus, les extrémités de raccordement 51 sont de préférence complémentaires les unes des autres de manière à former une section circulaire lorsque les extrémités de raccordement 51 sont disposées en contact les unes par rapport aux autres. De préférence, la section circulaire formée par les extrémités de raccordement 51 est inférieure au diamètre interne de la bague de raccordement 6. A titre d'exemple, lorsque le dispositif d'ancrage 4 comporte deux organes d'ancrage 5, les organes d'ancrage 5 comportent une extrémité de raccordement 51 de section hémicirculaire. A titre d'exemple encore, lorsque le dispositif d'ancrage 4 comporte trois organes d'ancrage 5, les organes d'ancrage 5 comportent une extrémité de raccordement 51 dont la section forme un secteur angulaire de 120°. Chaque extrémité de raccordement 51 comporte une surface extérieure 511 au contact de la bague de raccordement 6 et au moins une surface intérieure 512 disposée au contact d'au moins une autre extrémité de raccordement 51. La surface extérieure 511 est de préférence courbe et la surface intérieure 512 est de préférence plane. Le caractère extérieur ou intérieur des surfaces extérieure 511 et intérieure 512 est défini en considérant l'organe d'ancrage 5 lorsqu'il est disposé à l'intérieur de l'aiguille creuse de positionnement 2. Pour améliorer la tenue des organes d'ancrage 5 entre eux à l'intérieur de la bague de raccordement 6, la surface intérieure 512 peut comporter un relief, apporté par exemple par une rugosité de la surface, des rainures, des nervures ou des crans. De préférence, chaque organe de d'ancrage 5 comporte une extrémité de raccordement 51 identique. De manière alternative, chaque extrémité de raccordement 51 peut être différente, notamment en formant un secteur angulaire de valeur différente. A titre d'exemple, avec deux organes d'ancrage 5, l'une des extrémités de raccordement 51 peut présenter un secteur angulaire inférieur à 180° et l'autre des extrémités de raccordement 51 peut présenter un secteur angulaire supérieur à 180°. Les organes d'ancrage 5 sont de préférence assemblés dans la bague de raccordement 6 par sertissage, collage, soudure ou par écrasement de la bague de raccordement 5 sur les extrémités de raccordement 51.

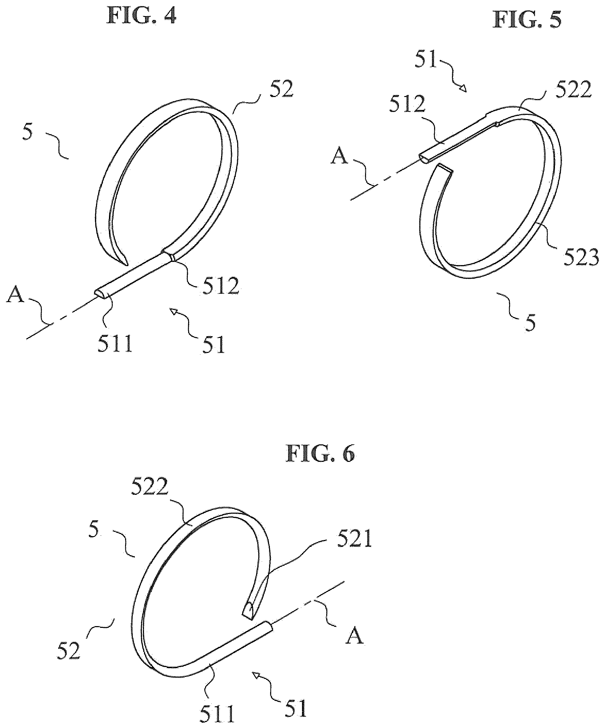

L'organe d'ancrage 5 comporte également une portion d'ancrage 52 formée dans le prolongement de l'extrémité de raccordement 51. La portion d'ancrage 52 comprend au moins une surface plane s'étendant transversalement à l'axe longitudinal A pour améliorer la capacité de l'organe d'ancrage 5 à être retenu dans les tissus biologiques. Ainsi, cette surface plane permet un ancrage efficace de l'organe d'ancrage 5 dans les tissus de sorte que les risques d'une migration du dispositif d'ancrage 4 est réduite. La portion d'ancrage 52 comporte également une surface externe 522 et une surface interne 523. Le caractère externe ou interne des surfaces interne 523 et externe 522 est défini en considérant l'organe d'ancrage 5 lorsqu'il est sorti hors de l'aiguille creuse de positionnement 2. De préférence, la surface plane est formée par la surface externe 522. De manière encore préférée, la surface externe 522 est réalisée dans le prolongement de la surface intérieure 512 de l'extrémité de raccordement 51. Cette portion d'ancrage 52 est de préférence courbée lorsque l'organe d'ancrage 5 est dans la deuxième position de manière à former un crochet et ainsi améliorer l'ancrage de l'organe d'ancrage 5. Selon le mode de réalisation représenté aux figures 4 et 5, au moins l'un des organes d'ancrage 5 a une section transversale formant un parallélogramme, de préférence un rectangle. Par exemple, la portion d'ancrage 52 peut être obtenue à partir d'une forme hémicirculaire aplatie. L'épaisseur de la portion d'ancrage 52 suivant une direction transversale à l'axe longitudinal A est choisie en fonction de la tenue au déploiement que l'on veut donner à la portion d'ancrage 52. Lorsque le dispositif d'ancrage 4 comporte deux organes d'ancrage 5, cette épaisseur est égale ou inférieure au demi-diamètre interne de l'aiguille creuse de positionnement 2. Toutes les formes comprises dans ce demi-diamètre sont autorisées. Suivant une forme préférentielle de réalisation, la forme déployée de l'organe d'ancrage 5 est réalisée par estampage d'un axe cylindrique dont le diamètre est préalablement défini afin d'obtenir les différentes sections.

Les organes d'ancrage 5 comportent en outre au niveau d'une extrémité opposée à l'extrémité de raccordement 51 un bord d'attaque 521 du tissu biologique formé par au moins une facette ou biseau. Cette facette ou biseau est par exemple formée par la portion d'ancrage 52. La facette ou le biseau principale de cet affûtage est réalisé sur la surface interne de l'organe d'ancrage 5 de façon favoriser le glissement de l'organe d'ancrage 5 lorsqu'il est éjecté de l'aiguille creuse de positionnement 2. Ce bord d'attaque 521 permet aussi aux organes d'ancrage 5 de perforer les tissus biologiques pour mettre en place le dispositif d'ancrage 4 en position d'ancrage.

Selon la figure 6, les organes d'ancrage 5 peuvent être réalisés suivant un autre mode de réalisation dans lequel la portion d'ancrage 52 a une section transversale hémicirculaire. De manière préférée, les organes d'ancrage 5 ont une section hémicirculaire continue. En d'autres termes, l'extrémité de raccordement 51 et la portion d'ancrage 52 peuvent avoir une section transversale hémicirculaire. De cette façon, lorsque le dispositif d'ancrage 4 comprend deux organes d'ancrage 5, ceux-ci peuvent être formés par une tige de section hémicirculaire dont une extrémité forme l'extrémité de raccordement 51, le reste étant formé par la portion d'ancrage 52. Le mode de réalisation de la figure 6 permet d'obtenir un organe d'ancrage 5 économique obtenu avec peu d'opérations de fabrication tout en garantissant une bonne retenue da l'organe d'ancrage 5 dans les tissus biologiques avec une surface externe 522 plane. L'organe d'ancrage selon la mode de réalisation de la figure 6 diffère du mode de réalisation des figures 4 et 5 en ce que la section transversale de la portion d'ancrage 52 est hémicirculaire et réalisée dans le prolongement de l'extrémité de raccordement 51. Toutefois, l'ensemble des autres caractéristiques décrites pour l'organe d'ancrage 5 des figures 4 et 5 sont compatibles avec le mode de réalisation de la figure 6. Bien entendu, lorsque le dispositif d'ancrage 4 comprend trois organes d'ancrage 5 selon le mode de réalisation de la figure 6, la section transversale de la portion d'ancrage 52 et de l'extrémité de raccordement 51 forment une portion angulaire correspondant à un tiers de cercle.

Tel qu'illustré sur les figures 4 à 6, l'extrémité opposée à l'extrémité de raccordement 51 forme une extrémité libre, i.e. elle n'est pas reliée ou solidaire de l'extrémité de raccordement 51. Cette extrémité opposée à l'extrémité de raccordement 51 est configurée à perforer des tissus biologiques, notamment par la présence du bord d'attaque 521.

Il est également proposé un premier dispositif d'ancrage alternatif selon les items suivants :
Item 1 : Dispositif d'ancrage pour le repérage de lésion ou de microcalcification dans un tissu biologique, comprenant :
   - un fil guide comportant au moins une portion graduée pour la détermination de la profondeur d'ancrage du dispositif d'ancrage,
   - au moins deux organes d'ancrage dans un tissu biologique raccordés au fil guide.
Item 2 : Dispositif d'ancrage selon l'item 1, dans lequel la portion graduée est plane.
Item 3 : Dispositif d'ancrage selon l'item 1 ou 2, dans lequel la portion graduée est obtenue par aplatissement du fil guide.
Item 4 : Dispositif d'ancrage selon l'un quelconque des items précédents, comprenant en outre une butée formant un appui pour l'entrainement longitudinal du dispositif d'ancrage.
Item 5 : Dispositif d'ancrage selon l'item 4, dans lequel la portion graduée comprend un élargissement local de la section transversale du fil formant la butée.
Item 6 : Dispositif d'ancrage selon l'un des items précédents, comprenant une bague de raccordement du fil guide aux organes d'ancrage, dans lequel la portion graduée est disposée à une longueur prédéterminée de la bague de raccordement.
Item 7 : Dispositif d'ancrage selon l'un des items précédents, dans lequel le fil guide est réalisé dans un matériau souple, de préférence en polyamide biocompatible.

Par ailleurs, il est également proposé un deuxième dispositif d'ancrage alternatif selon les items suivants :
Item 1 : Dispositif d'ancrage pour le repérage de lésion ou de microcalcification dans un tissu biologique, comprenant :
   - un fil guide,
   - au moins deux organes d'ancrage dans un tissu biologique raccordés au fil guide, chaque organe d'ancrage comporte une extrémité de raccordement s'étendant le long d'un axe longitudinal, les organes d'ancrage comprenant une surface de retenue plane s'étendant transversalement à l'axe longitudinal.
Item 2 : Dispositif d'ancrage selon l'item 1, dans lequel au moins l'un des organes d'ancrage a une section transversale formant un parallélogramme, de préférence un rectangle.
Item 3 : Dispositif d'ancrage selon l'item 1 ou 2, dans lequel les organes d'ancrage comprennent au niveau d'une extrémité opposée à l'extrémité de raccordement un bord d'attaque du tissu biologique formé par au moins deux facettes.

Par ailleurs, il est également proposé un troisième dispositif d'ancrage alternatif selon les items suivants :
Item 1 : Dispositif d'ancrage pour le repérage de lésion ou de microcalcification dans un tissu biologique, comprenant :
   - un fil guide,
   - au moins deux organes d'ancrage dans un tissu biologique raccordés au fil guide, et
   - une bague de raccordement du fil guide aux organes d'ancrage, dans lequel chaque organe d'ancrage comporte une extrémité de raccordement s'étendant le long d'un axe longitudinal et configurée pour être disposée à l'intérieur de la bague de raccordement, chaque extrémité de raccordement ayant une section transversale formant un secteur angulaire, la somme des secteurs angulaires étant égale ou inférieure à 360° dans un même plan transversal à la l'axe longitudinal.
Item 2 : Dispositif d'ancrage (4) selon l'item 1, dans lequel les extrémités de raccordement (51) sont complémentaires les unes des autres de manière à former une section circulaire lorsque les extrémités de raccordement (51) sont disposées en contact les unes par rapport aux autres.
Item 3 : Dispositif d'ancrage (4) selon l'item 1 ou 2, dans lequel chaque extrémité de raccordement (51) comporte une surface extérieure (511) au contact de la bague de raccordement (6) et au moins une surface intérieure (512) disposée au contact d'au moins une autre extrémité de raccordement (51).
Item 4 : Dispositif d'ancrage (4) selon l'item 3, dans lequel la surface extérieure (511) est courbe et la surface intérieure (512) est plane.
Item 5 : Dispositif d'ancrage (4) selon l'une quelconque des items précédents, dans lequel les organes d'ancrage (5) comprennent une portion d'ancrage (52) formée dans le prolongement de l'extrémité de raccordement (51), la portion d'ancrage (52) comprenant au moins une surface plane s'étendant transversalement à l'axe longitudinal (A).
Item 6 : Dispositif d'ancrage (4) selon l'une quelconque des items précédents, dans lequel les organes d'ancrage (5) comprennent au niveau d'une extrémité opposée à l'extrémité de raccordement (51) un bord d'attaque (521) du tissu biologique formé par au moins une facette.
Item 7 : Dispositif d'ancrage (4) selon l'une quelconque des items précédents, dans lequel chaque organe d'ancrage (5) est configuré pour être disposé dans :
   - une première position dans laquelle l'organe d'ancrage (5) a une configuration permettant d'insérer l'organe d'ancrage (5) dans un tissu biologique, et
   - une deuxième position dans laquelle l'organe d'ancrage (5) a une configuration permettant de retenir l'organe d'ancrage (5) dans un tissu biologique.
Item 8 : Dispositif d'ancrage (4) selon l'item 7, dans lequel les organes d'ancrage (5) sont configurés pour s'étendre dans des directions différentes les uns des autres lorsque les organes d'ancrage (5) sont chacun disposés dans leur deuxième position.

Les premier, deuxième et troisième dispositifs d'ancrage alternatifs peuvent aussi comporter une ou plusieurs des caractéristiques décrites dans la présente description prises dans toute combinaison techniquement admissible.

Il est à noter que chacun des dispositifs d'ancrage alternatifs présentés selon les items précédents est configuré pour être mis en oeuvre dans un système d'ancrage tel que décrit ci-avant et compatible avec l'ensemble des caractéristiques du dispositif d'ancrage 4 présenté ci-avant.

## Revendications

1. Dispositif d'ancrage (4) pour le repérage de lésion ou de microcalcification dans un tissu biologique, comprenant :
- un fil guide (7) comportant au moins une portion graduée (72) pour la détermination de la profondeur d'ancrage du dispositif d'ancrage (4),
- au moins deux organes d'ancrage (5) dans un tissu biologique raccordés au fil guide (7), et
- une bague de raccordement (6) du fil guide (7) aux organes d'ancrage (5).

2. Dispositif d'ancrage (4) selon la revendication 1, dans lequel la portion graduée (72) est plane.

3. Dispositif d'ancrage (4) selon la revendication 1 ou 2, dans lequel le fil guide (7) comprend une butée (75) formant un appui pour l'entrainement longitudinal du dispositif d'ancrage (4) par un mandrin.

4. Dispositif d'ancrage (4) selon la revendication 13, dans lequel la portion graduée (72) comprend un élargissement local de la section transversale du fil formant la butée (75).

5. Dispositif d'ancrage (4) selon l'une quelconque des revendications, dans lequel la portion graduée (72) est disposée à une longueur prédéterminée de la bague de raccordement (6).

6. Dispositif d'ancrage (4) selon l'une quelconque des revendications, dans lequel le fil guide (7) est réalisé dans un matériau souple, de préférence en polyamide biocompatible.

7. Dispositif d'ancrage (4) selon l'une quelconque des revendications, dans lequel le fil guide (7) comporte en outre :
- une portion proximale (73) destinée à être sertie dans la bague de raccordement (6), le diamètre externe de la portion proximale (73) étant inférieur à une dimension transversal maximale de la portion graduée (72), et
- une portion distale (71) configurée pour coulisser dans le corps d'un mandrin (31).

8. Dispositif d'ancrage (4) selon l'une quelconque des revendications précédentes, dans lequel chaque organe d'ancrage (5) comporte une extrémité de raccordement (51) s'étendant le long d'un axe longitudinal (A) et configurée pour être disposée à l'intérieur de la bague de raccordement (6), chaque extrémité de raccordement (51) ayant une section transversale formant un secteur angulaire, la somme des secteurs angulaires étant égale ou inférieure à 360° dans un même plan transversal à la l'axe longitudinal (A).

9. Dispositif d'ancrage (4) selon la revendication 8, dans lequel les extrémités de raccordement (51) sont complémentaires les unes des autres de manière à former une section circulaire lorsque les extrémités de raccordement (51) sont disposées en contact les unes par rapport aux autres.

10. Dispositif d'ancrage (4) selon la revendication 8 ou 9, dans lequel chaque extrémité de raccordement (51) comporte une surface extérieure (511) au contact de la bague de raccordement (6) et au moins une surface intérieure (512) disposée au contact d'au moins une autre extrémité de raccordement (51).

11. Dispositif d'ancrage (4) selon la revendication 10, dans lequel la surface extérieure (511) est courbe et la surface intérieure (512) est plane.

12. Dispositif d'ancrage (4) selon l'une quelconque des revendications précédentes, dans lequel les organes d'ancrage (5) comprennent une portion d'ancrage (52) formée dans le prolongement de l'extrémité de raccordement (51), la portion d'ancrage (52) comprenant au moins une surface plane s'étendant transversalement à l'axe longitudinal (A).

13. Dispositif d'ancrage (4) selon la revendication 12, dans lequel a portion d'ancrage (52) comporte une surface externe (522) plane et une surface interne (523) courbe.

14. Dispositif d'ancrage (4) selon l'une quelconque des revendications précédentes, dans lequel les organes d'ancrage (5) comprennent au niveau d'une extrémité opposée à l'extrémité de raccordement (51) un bord d'attaque (521) du tissu biologique formé par au moins une facette.

15. Dispositif d'ancrage (4) selon l'une quelconque des revendications précédentes, dans lequel chaque organe d'ancrage (5) est configuré pour être disposé dans :
- une première position dans laquelle l'organe d'ancrage (5) a une configuration permettant d'insérer l'organe d'ancrage (5) dans un tissu biologique, et
- une deuxième position dans laquelle l'organe d'ancrage (5) a une configuration permettant de retenir l'organe d'ancrage (5) dans un tissu biologique.

17. Système d'ancrage (10) pour le repérage de lésion ou de microcalcification dans un tissu biologique, comprenant :
- une aiguille creuse de positionnement (2),
- un dispositif d'ancrage (4) selon l'une quelconque des revendications précédentes configuré pour coulisser à l'intérieur de l'aiguille creuse de positionnement (2),
- un mandrin (3) configuré pour coulisser à l'intérieur de l'aiguille creuse de positionnement (2) de manière à entrainer les organes d'ancrage (5) hors de l'aiguille creuse de positionnement (2).

18. Système d'ancrage (10) selon la revendication 17, dans laquelle l'un au moins parmi les deux organes d'ancrage (5) du dispositif d'ancrage (4) comporte une section transversale égale ou inférieure au demi-diamètre interne de l'aiguille creuse de positionnement (2).

19. Système d'ancrage (10) selon la revendication 17 ou 18, dans lequel le mandrin (3) comporte un trou traversant permettant au mandrin (3) de coulisser autour et le long du fil guide (7) du dispositif d'ancrage (4).

20. Système d'ancrage (10) selon l'une des revendications 17 à 19 en combinaison avec le dispositif d'ancrage (4) selon la revendication 15, dans lequel l'aiguille creuse de positionnement (2) est configurée pour disposer les organes d'ancrage (5) dans la première position lorsque les organes d'ancrage (5) sont disposés à l'intérieur de l'aiguille creuse de positionnement (2), les organes d'ancrage (5) étant configurés pour se déformer en vue d'adopter la deuxième position une fois entrainés hors de l'aiguille creuse de positionnement (2).
